(19) Europäisches Patentamt
European Patent Office
Office européen des brevets

(11) **EP 3 975 826 B1**

(12) **EUROPEAN PATENT SPECIFICATION**

(45) Date of publication and mention
of the grant of the patent:
**26.06.2024  Bulletin 2024/26**

(21) Application number: **20730483.3**

(22) Date of filing: **14.05.2020**

(51) International Patent Classification (IPC):
***A61B 5/00*** *(2006.01)*    ***A61B 5/11*** *(2006.01)*

(52) Cooperative Patent Classification (CPC):
**A61B 5/4827; A61B 5/0051; A61B 5/1101;**
A61B 2562/0219

(86) International application number:
**PCT/IB2020/054579**

(87) International publication number:
**WO 2020/245681 (10.12.2020 Gazette 2020/50)**

(54) **SYSTEMS FOR ASSESSMENT OF HAPTIC PERCEPTION IMPAIRMENTS AND MOTOR CONTROL ABNORMALITIES**

SYSTEME ZUR BEURTEILUNG VON HAPTISCHEN WAHRNEHMUNGSBEEINTRÄCHTIGUNGEN UND MOTORIKANOMALIEN

SYSTÈMES D'ÉVALUATION DE DÉFICIENCES DE PERCEPTION HAPTIQUE ET D'ANOMALIES DE MOTRICITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **03.06.2019  US 201962856459 P**

(43) Date of publication of application:
**06.04.2022  Bulletin 2022/14**

(73) Proprietor: **3M Innovative Properties Company**
**Saint Paul, Minnesota 55133-3427 (US)**

(72) Inventors:
• **KAHL, Jonathan T.**
**Saint Paul, Minnesota 55133-3427 (US)**
• **KNUDSON, Orlin B.**
**Saint Paul, Minnesota 55133-3427 (US)**
• **ANDERSON, Nathan J.**
**Saint Paul, Minnesota 55133-3427 (US)**
• **KAPPEL, Glendon D.**
**Saint Paul, Minnesota 55133-3427 (US)**
• **KENDRICK, Paul A.**
**Saint Paul, Minnesota 55133-3427 (US)**
• **ANDERSON, Nathaniel D.**
**Saint Paul, Minnesota 55133-3427 (US)**

(74) Representative: **Vossius & Partner**
**Patentanwälte Rechtsanwälte mbB**
**Siebertstraße 4**
**81675 München (DE)**

(56) References cited:
**US-A- 5 002 065     US-A1- 2012 220 892**

• **GOLDBERG J M ET AL: "Standardised method of detemining vibratory perception thresholds for diagnosis and screening in neurological investigation", JOURNAL OF NEUROLOGY NEUROSURGERY & PSYCHIATRY, BMJ PUBLISHING GROUP, GB, vol. 42, no. 9, 1 September 1979 (1979-09-01), pages 793-803, XP008123751, ISSN: 0022-3050**
• **WENTINK E C ET AL: "Vibrotactile stimulation of the upper leg: Effects of location, stimulation method and habituation", ENGINEERING IN MEDICINE AND BIOLOGY SOCIETY,EMBC, 2011 ANNUAL INTERNATIONAL CONFERENCE OF THE IEEE, IEEE, 30 August 2011 (2011-08-30), pages 1668-1671, XP032319053, DOI: 10.1109/IEMBS.2011.6090480 ISBN: 978-1-4244-4121-1**

- E.M. JÖBGES ET AL: "Vibratory proprioceptive stimulation affects Parkinsonian tremor", PARKINSONISM AND RELATED DISORDERS, vol. 8, no. 3, 1 January 2002 (2002-01-01) , pages 171-176, XP055718522, GB ISSN: 1353-8020, DOI: 10.1016/S1353-8020(01)00016-5
- RIBOT-CISCAR EDITH ET AL: "Sensory training with vibration-induced kinesthetic illusions improves proprioceptive integration in patients with Parkinson's disease", JOURNAL OF NEUROLOGICAL SCIENCES, ELSEVIER SCIENTIFIC PUBLISHING CO, AMSTERDAM, NL, vol. 383, 20 November 2017 (2017-11-20), pages 161-165, XP085305322, ISSN: 0022-510X, DOI: 10.1016/J.JNS.2017.11.016

## Description

## Background

Degradation of touch sensitivity is a common result of disease, injury and aging. However, the scientific understanding of the underlying neural mechanisms that cause these degradations is poorly understood. Quantitative monitoring of touch sensitivity over periods of time can provide valuable information for research on therapy and disease assessment. In addition, there are several diseases and conditions that selectively impair the motor system of the nervous system. Tremor and dystonia are a few motor impairments/abnormalities that result from disease, injury, and certain prescription medications. A better understanding of tremors, and earlier detection of low-level tremors, can improve patient outcomes. US 2012/220892 A1 discloses a vibrometer capable of applying vibrotactile stimulation to a test subject's skin to determine the existence of peripheral neuropathy. US 5 002 065 A discloses an apparatus for measuring sensory disturbances of a patient by applying normalized vibrator forces to a body portion of the patient, automatically effecting discrete vibrations of the body portion, noting the onset of sensory perception by the patient and correlating the information with the physical condition of the body portion. GOLDBERG J M ET AL "Standardised method of determining vibratory perception thresholds for diagnosis and screening in neurological investigation" (JOURNAL OF NEUROLOGY NEUROSURGERY & PSYCHIATRY, BMJ PUBLISHING GROUP, GB vol 42, no. 9, 1 September 1979) discloses an analysis in which vibration threshold determinations were made by means of an electromagnetic vibrator at three sites of a patient to check for impairment of vibratory sensibility.

## Summary

The present invention is defined by the features of the independent claim(s). Preferred embodiments are defined by the features of the dependent claims. In broad summary, herein are disclosed systems, apparatuses and methods for measuring tremors and peripheral nerve sensitivity. An apparatus can include a housing and an actuator and/or accelerometer accessible from an outer surface of the housing. An actuator can generate a stimulation signal to a skin surface of a subject. Processing circuitry can control the actuator to generate a stimulation signal, and record response to the stimulation signal to determine vibrotactile sensitivity. If an accelerometer is used, vibration generated by the subject can be measured. These and other aspects will be apparent from the detailed description below.

## Brief Description of the Drawings

FIG. 1 is a diagram of a system for assessment of haptic perception impairments and motor control abnormalities according to an example embodiment.

FIG. 2 is a diagram of a system including distributed modules for assessment of haptic perception impairments and motor control abnormalities according to an example embodiment.

FIG. 3 illustrates an outside surface of an actuator assembly that can be included in a system for assessment of haptic perception impairments and motor control abnormalities in accordance with some embodiments.

FIG. 4 is a hidden line view of an actuator assembly that can be included in a system for assessment of haptic perception impairments and motor control abnormalities in accordance with some embodiments.

FIG. 5 illustrates a cutaway view of an actuator assembly in accordance with some embodiments.

FIG. 6 is a block diagram showing placement of a mechanically compliant window in contact with an actuator in accordance with some embodiments.

FIG. 7 is a partial block diagram showing placement of an infection barrier on a surface of an actuator assembly in accordance with some embodiments.

FIG. 8 is a block diagram depicting components of an actuator assembly in accordance with some embodiments.

FIG. 9 illustrates a flowchart of a method of measuring vibrotactile perception according to some embodiments.

FIG. 10 illustrates a flowchart of a method detecting tremors indicative of a neurological disorder according to some embodiments.

FIG. 11 illustrates thresholds for vibration detection on a human subject.

FIG. 12 illustrates measurements taken at different times on a human subject.

FIG. 13 illustrates signal flow from a vibration capture according to some embodiments.

FIG. 14 illustrates a computing system upon which some example embodiments may be implemented.

**Detailed Description**

In the following description, reference is made to the accompanying drawings that form a part hereof, and in which is shown by way of illustration specific embodiments which may be practiced. These embodiments are described in sufficient detail to enable those skilled in the art to practice the invention, and it is to be understood that other embodiments may be utilized and that structural, logical and electrical changes may be made without departing from the scope of the present invention. The following description of example embodiments is, therefore, not to be taken in a limited sense, and the scope of the present invention is defined by the appended claims.

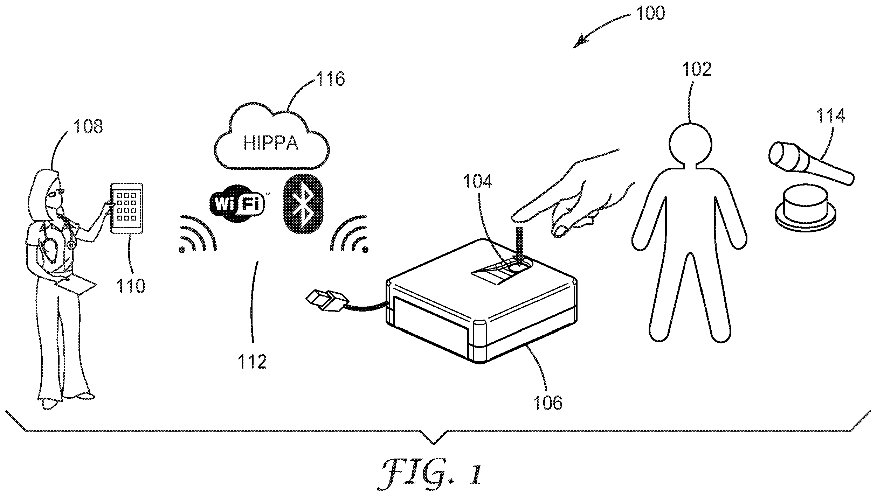

Haptic perception impairments and motor control abnormalities can be diagnosed and assessed in a clinical environment. FIG. 1 is a diagram of a system 100 for assessment of haptic perception impairments and motor control abnormalities according to an example embodiment. As depicted in FIG. 1, a subject 102 may make contact (e.g., by placing a finger) with a mechanism (e.g., vibrotactile actuator 104) of an apparatus, for example an actuator assembly 106. Under the control of the clinician 108 or using a prefigured test protocol, a clinician device 110 (e.g., a PC, laptop, tablet, or smart phone, henceforth referred to as PDA)) can communicate over a wired or wireless interface 112 to direct the actuator assembly 106 to send vibrotactile stimulation/s to a skin surface of the subject 102. In some examples, the skin surface includes a fingertip, although embodiments are not limited thereto. The vibrotactile stimulation/s may be a single pulse, or a series of stimulations based on standard benchmarks or test protocols, or custom specific instruction/s from the clinician 108.

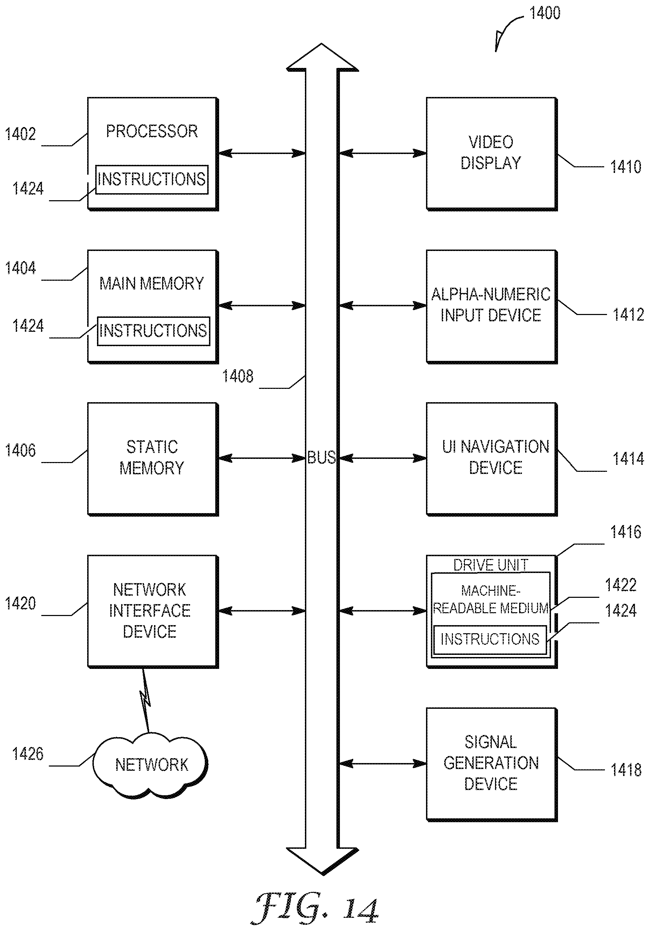

The subject 102 can provide feedback using a feedback device 114 acknowledging that the stimulus has been perceived. Feedback can be verbal, via computer screen button, touch screen button, dedicated button, keyboard, etc. The system 100 stores data (time of test, frequency and amplitude of stimulations, patient feedback, etc.), for analysis. This can be stored in the cloud 116, at the clinician device 110, or within the actuator assembly 106 itself, in a memory as described with respect to FIG. 14, or in other storage or a combination of other storage.

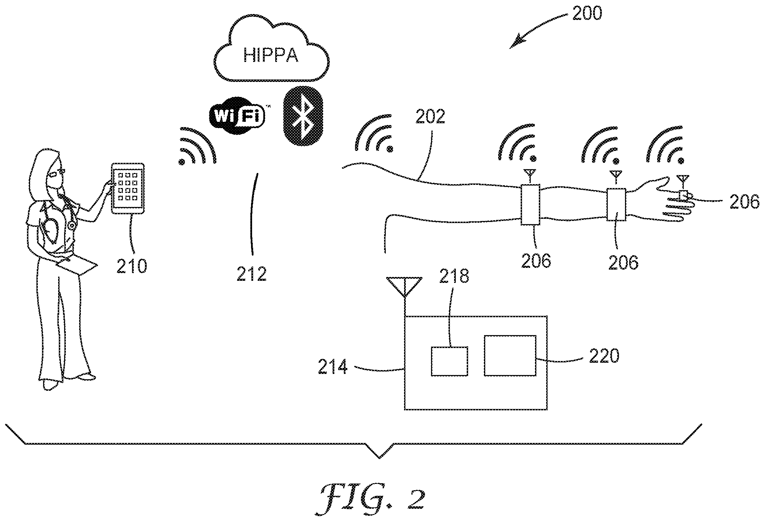

FIG. 2 is a diagram of a system 200 including distributed actuator assemblies 206 for assessment of haptic perception impairments and motor control abnormalities according to an example embodiment. The subject 202 can have actuator assemblies 206 distributed along a limb as shown, or on multiple limbs or other skin surfaces or locations on the subject 202. The multiple actuator assemblies 206 communicate over a wired or wireless interface 212 amongst each other, or to the clinician device 210. In some aspects, multiple actuator assemblies 206 can communicate wirelessly or over a wired connection to each other through a central controller 214 that can additionally include antenna/s 216, communication circuitry 218 and processing circuitry 220 for control of such communication.

Example apparatuses

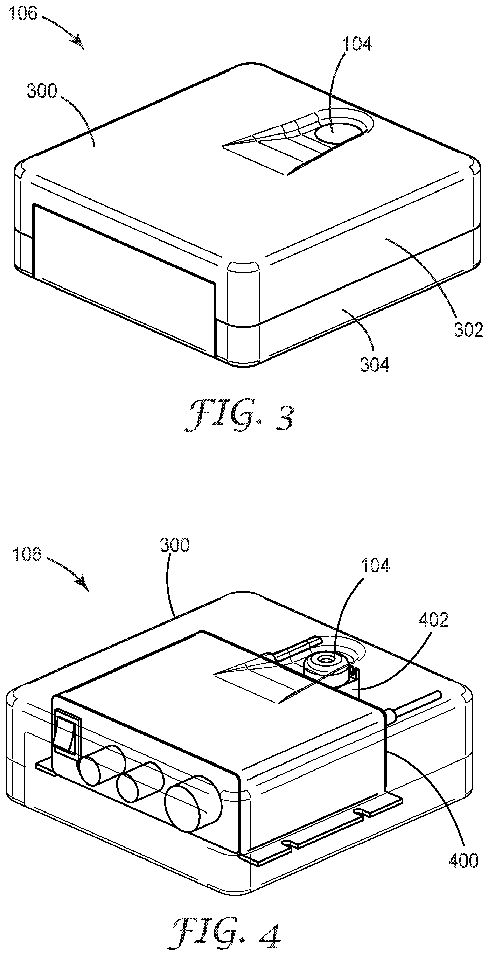

FIG. 3 illustrates an outside surface of an actuator assembly 106 that can be included in a system (e.g., system 100 (FIG. 1) or system 200 (FIG. 2)) for assessment of haptic perception impairments and motor control abnormalities in accordance with some embodiments. The vibrotactile actuator 104 can be uncovered and exposed as shown, although embodiments are not limited thereto. A housing 300 can be provided. In embodiments, the actuator 104 is accessible from an outer surface of the housing 300 and configured to generate a stimulation signal (e.g., a vibration) to a skin surface of a subject 102 (FIG. 1).

The housing 300 can include a top piece 302 and a bottom piece 304 joined together, although embodiments are not limited thereto. Materials with resistance to cleaning chemicals can be used to construct the housing 300. Such materials can include one or more thermoplastic material types, such as polypropylene or acrylonitrile butadiene styrene (ABS), although embodiments are not limited thereto. Pieces (e.g., top piece 302 and bottom piece 304) should couple sufficiently to prevent water droplets from forming on internal electrical components when subjected to moderate spraying of cleaning solutions. A mechanism or apparatus (for example, an elastic strap or band, or an adhesive not shown in FIG. 3) can be provided to secure a body part (e.g., digit, limb or portion thereof, upper or lower extremities, face or head) of the subject 102 such that the relevant body part can be provided or stimulated with a controllable and known baseline normal force upon the vibrotactile actuator 104.

The vibrotactile actuator 104 surface can be contoured for finger, hand or foot comfortable and repeatable placement. Vibration dampening in the form of ballast, sprung mass or shock absorptive mounts may be used to isolate the device from ambient vibration. The assembly 106 and/or housing 300 can be contoured to fit into or against, for example, the palm or other body part of the subject 102. The actuator 104 may induce vibration at the skin surface being stimulated. The vibration may be normal, or nearly normal to the skin surface although embodiments are not limited thereto. The actuator 104 may induce vibrations of known frequency, amplitude and phase through control using processing circuitry or other circuitry described later herein. The actuator 104 may comprise a piezoelectric disk (available from, for example, Steiner & Martins, Inc., of Dolan, Florida); voice coil; eccentric rotating mass (ERM) actuator (available from Precision Microdrives of London, England), or other type of actuator.

FIG. 4 is a hidden line view of an actuator assembly 106 that can be included in a system for assessment of haptic perception impairments and motor control abnormalities in accordance with some embodiments. As shown in FIG. 4, circuitry within the actuator assembly 106 can include an amplifier 400 for amplifying signals to or from the vibrotactile actuator, and a normal force sensor 402 for sensing downward force at the vibrotactile actuator 104. Output (e.g., analog or digital output) of the normal force sensor 402 can be displayed or output to other computing systems of system 100 as described in further detail later herein. Other circuitry and structures can be included.

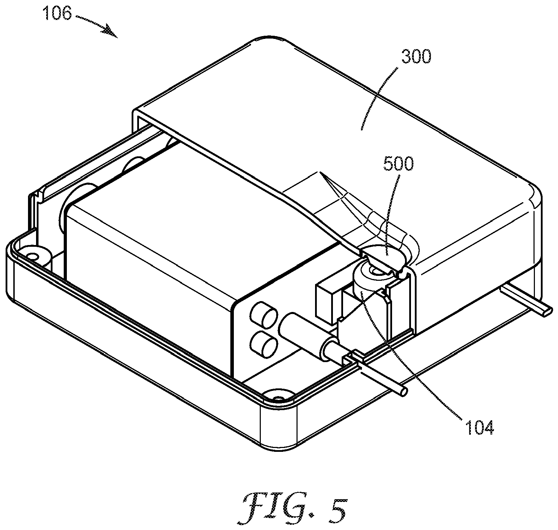

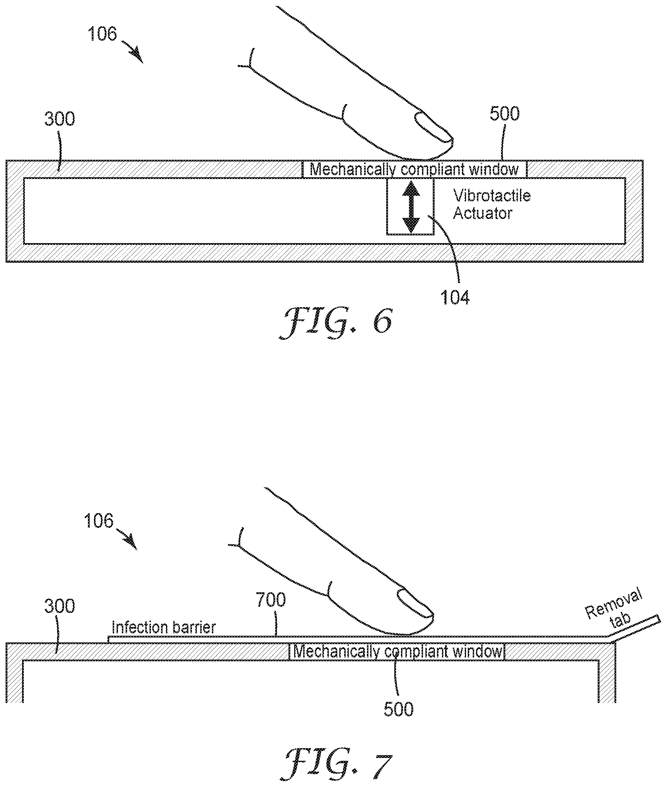

In some examples, gaps and seams in the housing 300 (FIG. 3 and FIG. 4) can be minimized or eliminated to prevent infection, to prevent damage to circuitry within the housing 300, or for any other reason. Gaps and seams can be reduced or eliminated by providing a section within the housing 300 comprised of a having a mechanically compliant surface proximate (e.g., in contact with, such that the actuator 104 is able to move the compliant surface 500) the actuator 104. This is illustrated in FIG. 5 and FIG. 6. FIG. 5 illustrates a cutaway view of an actuator assembly 106. As shown in FIG. 5, a section of housing 300 can comprise a mechanically compliant surface 500. As shown, the mechanically compliant surface 500 can be in contact with the actuator 104.

In examples, the mechanically compliant surface 500 facilitates transference of vibrations between the actuator 104 and a skin surface, digit, etc., of the subject 102. In some examples, the mechanically compliant surface 500 can be created by molding a thermoplastic urethane (TPU) or silicone membrane over the top of housing 300. In some embodiments, the mechanically compliant surface 500 can encompass the seam joining top piece 302 (FIG. 3) and bottom piece 304 (FIG. 3) to create an integrated gasket.

A separate piece of formed compliant material may be used to snap into recesses in the top of the housing 300 in some embodiments. In some embodiments, a flat piece of compliant material such as nitrile material may be clamped over the top of the actuator 104. A stiff piece of material may be used as a piston to transfer vibrations. In this case a separate mechanically complaint surface 500 could be used to insure the housing 300 stays resistant to water incursion.

FIG. 6 is a block diagram showing placement of a mechanically compliant surface 500 in accordance with some embodiments. As shown, a skin surface (e.g.,, fingertip) of a human subject can be presented to the mechanically compliant surface 500 to sense stimulation signals provided by the actuator 104.

An additional barrier to the spread of infection can include a disposable barrier between the subject and the actuator assembly. In aspects, these sterile barriers can be provided by a sterile dispensing system and disposed of after usage. FIG. 7 is a partial block diagram showing placement of an infection barrier 700 on a surface of an actuator assembly 106 in accordance with some embodiments. In examples, the infection barrier 700 is placed proximate, or at least partially overlapping, the mechanically compliant surface 500.

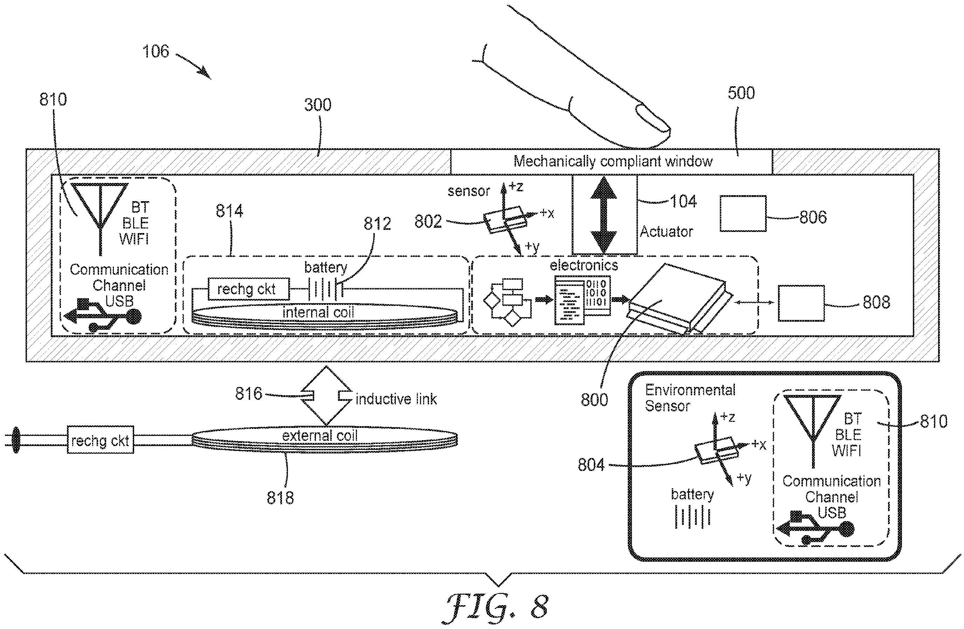

In addition to the components of an actuator assembly 106 already described herein, an actuator assembly 106 can include other components for providing control, communication, sensing, and other functionalities. FIG. 8 is a block diagram depicting components of an actuator assembly in accordance with some embodiments. In FIG. 8, components having counterparts in FIG. 1-7 are described using reference numerals corresponding to those used in FIG. 1-7.

Referring to FIG. 8, an apparatus (e.g., actuator assembly 106) includes housing 300, and an actuator 104 accessible from an outer surface of the housing 300. The actuator assembly 106 further includes processing circuitry 800. The processing circuitry 800 can control the actuator 104 to generate the above-described stimulation signal and record a response to the stimulation signal. The stimulation signal can be in an axis perpendicular to the mechanically compliant surface 500. In some embodiments (e.g., embodiments similar to those shown in FIG. 3 and FIG. 4), no mechanically compliant surface 500 is present, i.e., the finger or other body portion of the patient can directly contact the actuator 104.

The actuator assembly 106 can further include an accelerometer 802 to measure vibration. The accelerometer 802 can be a multi-axis accelerometer. An environmental sensor 804 can measure environmental vibration (building vibrations, bodily vibrations, etc.) or other vibrations distinct from the intended vibrotactile stimulus or patient tremor, and this can be used to mitigate the unwanted environmental vibrations by subtracting the vibration from the measured vibration of the actuator assembly 106. The actuator assembly 106 can further include a displacement sensor 806 to measure displacement of the mechanically compliant surface 500. The displacement sensor can be a laser displacement sensor. In some aspects, the displacement sensor can be a Doppler (LD) meter or sensor (for example, the Keyence LK-G5000 series Laser Displacement Sensor, available from Keyence of Itasca, Illinois, USA).

The actuator assembly 106 can further include a memory, depicted and described in more detail later herein with reference to FIG. 14. The processing circuitry 800 can store data, such as vibrations measured by the accelerometer 802, or other information including date information, time information, stimulation information (e.g., amplitude, frequency or phase of the vibration signal), protocol information, subject identification information, and subject response information.

The actuator assembly 106 can include other sensors and circuitry 808, including for example temperature sensors and humidity sensors, force sensors, and force control circuitry. Example temperature and humidity sensors can include sensors available from STMicroelectronics headquartered in Geneva, Switzerland. Example force sensors include FlexiForce sensors (available from TekScan of Boston, MA) or FX force sensors available from TE Connectivity headquartered in Schaffhausen, Switzerland. These and other sensors and control systems can provide analog or digital signals to the processing circuitry 800 using, for example any suitable bus such as universal serial bus (USB), I$^2$C or SPI serial buses. Force control circuitry can control the force applied by the subject to the actuator.

The actuator assembly 106 can include communication circuitry 810. Communication circuitry 810 can be used to communicate over a wired or wireless interface 112 (FIG. 1) to the cloud 116 (FIG. 1), to clinician device 110 (FIG. 1), to other actuator assemblies (e.g., in other actuator assemblies of system 200 (FIG. 2)), to remote storage, etc. Communication channels can be used for communication according to standards compliant with Wi-Fi, cellular, Bluetooth, USB, or other communication standards. Communications can include data streaming or periodic data uploads to or from the actuator assembly 106, software updates and downloads, etc.

The actuator assembly 106 can include a battery 812, which can be rechargeable, or power can be provided by a connection such as through a power jack or USB port. The battery (if present) can be recharged through an internal charging circuit 814 inductively coupled at 816 to an external coil 818.

Example methods

Referring again to FIG. 1 and FIG. 2, the systems 100 and 200 can operate in at least two modes. In a first mode, vibrotactile perception is measured by injecting vibration (e.g., providing a stimulation signal) to the subject 102, and measuring perceptual response. In a second mode, tremors are quantized according to a tremor quantization method to detect motor control abnormalities.

In the first (vibrotactile perception) mode the system 100 (or 200 for distributed system embodiments) determines the point at which a patient can detect a vibrotactile stimulus launched from the actuator assembly 106 and records patient perception. When taken over time changes in just-noticeable difference (JND) thresholds of perception can be quantified.

Such perception can occur at various receptors on the subject's body. There are four primary mechanoreceptors found in the glabrous (hairless) skin. Each of these mechanoreceptors respond to unique types of mechanical vibration. Each type of mechanoreceptor respects to different types of mechanical vibration, as shown in Table 1:

**Table 1: Mechanoreceptor sensing capabilities**

| Mechanoreceptor | Best at Sensing | Frequency range |
| --- | --- | --- |
| Merkel's Cells | Pressure (slower movements) | 0.4 - 100 Hz (5-15 Hz peak) |
| Ruffini Ending | Presser (slower movements) | 7 Hz |
| Meissner's Corpuscle | Touch (faster movements) vibration | 10-200 Hz (10-30 Hz peak) |
| Pacinian Corpuscle | Vibration | 40-800 Hz (200-300 Hz peak) |

In methods according to at least some embodiments, processing circuitry 800 can control the actuator 104 (through commands issued by clinician device 110, for example) to sweep through various frequency ranges and determine the sensitivity of the patient to each vibrotactile frequency range. As measurements are taken over time, researchers can reach a greater understanding about the progression of peripheral neuropathy and how the different mechanoreceptors are impacted. Impact of drug therapy can also be observed. Methods for measuring vibrotactile perception and for detecting tremors are described below.

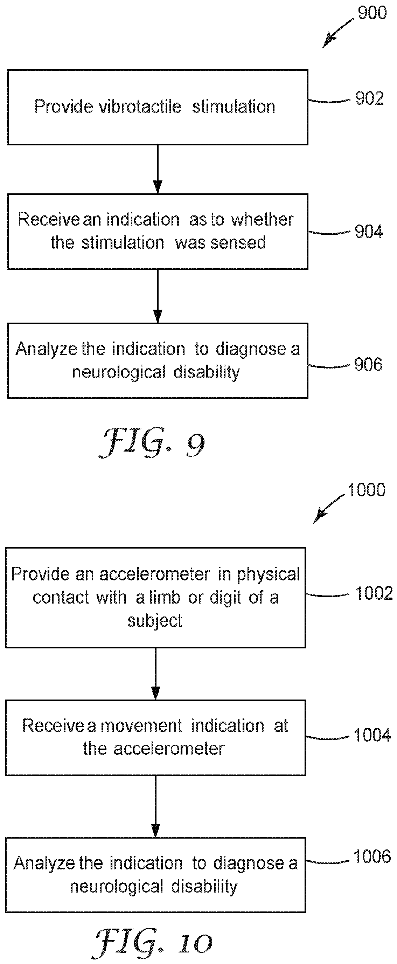

FIG. 9 illustrates a flowchart of a method 900 of measuring vibrotactile perception according to some embodiments. Operations of method 900 can be executed by processing circuitry 800 (FIG. 8), by elements of system 100 (FIG. 1) or system 200 (FIG. 2), or by any other processing circuitry or computing system.

Method 900 begins with operation 902 with the processing circuitry 800 providing vibrotactile stimulation to an actuator in physical contact with a skin surface of a subject. In some embodiments, the vibrotactile stimulation is a single pulse.

In other embodiments, the vibrotactile stimulation includes a series of pulses based on standard benchmarks or protocols. In some embodiments, at least one pulse has a frequency of between about .4 - 100 Hz to stimulate Merkel's receptors. In some embodiments, at least one pulse of has a frequency of about 7 Hz to stimulate a Ruffini corpuscle. In some embodiments, at least one pulse has a frequency of about 10-200 Hertz to stimulate a Meissner's corpuscle. In some embodiments, at least one pulse has a frequency of about 40-800 Hertz. In some embodiments, a series of pulses includes pulses from more than one frequency range.

Method 900 continues with operation 904 with the processing circuitry 800 receiving an indication as to whether the stimulation was sensed. As described with respect to FIG. 1, this indication can be provided through feedback device 114 (e.g., via verbal feedback, computer screen button, touch screen button, dedicated button, keyboard, etc.) In some embodiments, the processing circuitry 800 can store the indication in memory. The processing circuitry 800 can store other data including date information, time information, stimulation information, protocol information, subject identification information, and subject response information in the memory, whether same memory or other memory, wherein the memory is described in more detail below with reference to FIG. 14.

Method 900 continues with operation 906 with the processing circuitry 800 analyzing the indication to diagnose a neurological disability. In some embodiments, the analyzing can include comparing recorded information over time to detect a change in vibrotactile perception. Further description of the analysis is provided later herein.

In some embodiments, when a distributed system such as system 200 (FIG. 2) is used, the method 900 can include the processing circuitry 800 providing the vibrotactile stimulation to a plurality of actuators in physical contact at a plurality of points on the skin surface of the subject. In at least these embodiments, the processing circuitry can receive indications from the plurality of actuators and compare sensitivity at the plurality of points based on the indications. The processing circuitry 800 can provide this information to other elements in system 200 and analysis can be performed at these other elements (for example, clinician device 110, cloud 116, etc.) In some embodiments, operations of method 900 can be combined with operations of method 1000 described later below, to both measure vibrotactile perception and detect tremors.

FIG. 10 illustrates a flowchart of a method 1000 of detecting tremors indicative of a neurological disorder according to some embodiments. Operations of method 1000 can be executed by processing circuitry 800 (FIG. 8) or other element of the actuator assembly 106 (FIG. 1), by elements of system 100 (FIG. 1) or system 200 (FIG. 2), or by any other processing circuitry or computing system. In some scenarios in which vibrotactile stimulation is not measured, the actuator assembly 106 may include an accelerometer but no actuator and can be referred to as an accelerometer assembly.

Method 1000 begins with operation 1002 with providing an accelerometer (e.g., accelerometer 802 (FIG. 8) in physical contact with an upper or lower limb, digit, hand, or foot, face, etc. of a subject (e.g., subject 102 (FIG. 1). In some embodiments, a plurality of accelerometers 802 is provided by providing multiple actuator assemblies 206 as depicted in system 200 (FIG. 2). In these and other embodiments, a plurality of accelerometers is provided at multiple points on limbs or digits of the subject. Then, if location-based tremors are suspected, the processing circuitry 800 can compare movement indicators from the plurality of accelerometers to determine where tremors are generated or where tremors occur.

Method 1000 continues with operation 1004 with the processing circuitry 800 receiving a movement indication at the accelerometer 802.

Method 1000 continues with operation 1006 with the processing circuitry analyzing the indication to diagnose a neurological disability. In embodiments depicted according to FIG. 2, multiple indications from multiple actuator assemblies 206 are received and analyzed by the processing circuitry 800. In some embodiments, processing circuitry 800 can compare recorded information over time to detect a change in tremor severity or tremor frequency. In some embodiments, operations of method 1000 can be combined with operations of method 900 described earlier herein, to both measure vibrotactile perception and detect tremors.

Analysis such as that performed in methods similar to methods 900 and 1000 can include correlation of tremor statistics to posture and physical activity may give provide diagnostic information for the clinician. For example, processing circuitry 800 can record tremor acceleration data and break this raw data into frames of data. The processing circuitry 800 can analyze each frame for frequency, frequency variation, and intensity. The processing circuitry 800 can also perform other statistical analysis including average frequency, frequency standard deviation or coefficient of variation, and intensity. For example, a resting tremor may be indicative of Parkinson's (frequencies between 4-8 Hz), and this resting tremor can have a first frequency spectrum that can be analyzed. In Parkinson's a resting tremor may be temporarily reduced during activity only to return (called a re-emergent tremor); this can have a separate indicative frequency spectrum, distinct from the frequency spectrum indicating a resting tremor. Dystonic tremors are irregular and jerky, which produce a different frequency spectrum than the consistent Parkinson tremor. Methods similar to those described earlier herein (particularly with reference to FIG. 10) can provide quantitative data of this behavior to detect frequency spectra indicative of different types of tremors.

The displacement of the mechanically compliant surface of an actuator assembly described earlier herein can be used for motor impairment assessment of physical phenomena other than tremors. For instance, subject 102 (FIG. 1) can be informed to perform a goal directed movement on the actuator assembly 106 (FIG. 1) and various kinematics such as reaction time, movement accuracy, and velocity and acceleration can be assessed.

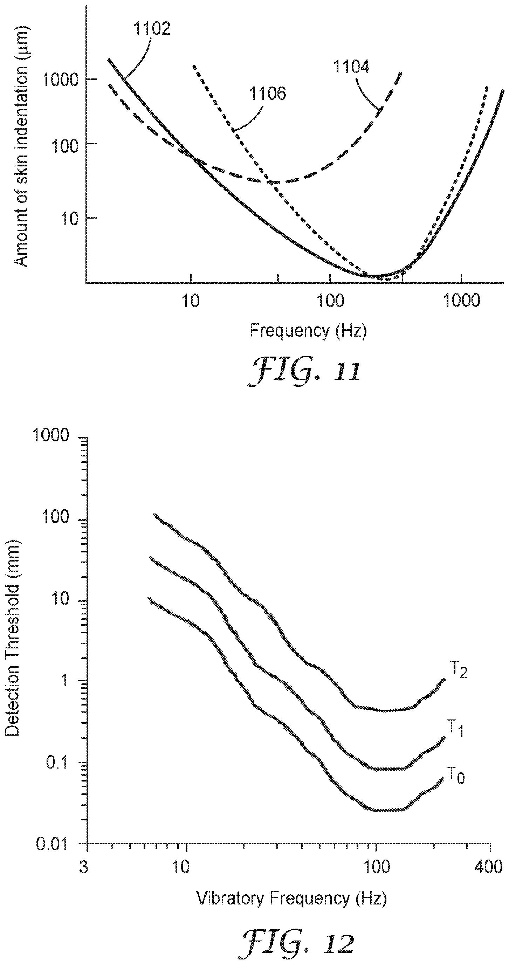

FIG. 11 illustrates thresholds for vibration detection on a human subject. In a setup similar to that shown in FIG. 1 and FIG. 2, assessment of haptic perception deficits can be set up based on typical detection thresholds shown in FIG. 11. For example, a threshold of human perception is shown at curve 1102. One example of experimental setup would be to have the subject 102 indicate whether a signal is present or absent during a stimulus trial, wherein the frequency of the stimulation signal and amount of skin indentation generated by such stimulation is as indicated according to curve 1102 (normal or unimpaired human detection threshold). In some stimulus trials the stimulus signal will be presented while in some trials the signal will be absent. For analysis, the researcher can quantify the haptic displacement threshold required, at any given vibrotactile frequency, for the participant to successful detect the presence of the stimulus. Detection of defects in Meissner's corpuscle or Pacinian corpuscle can be detected by comparing results against curves 1104 and 1106, respectively.

FIG. 12 illustrates measurements taken at different times on a human subject. Measurements taken at time T0 may be less indicative of disease, as sensitivity occurs at lower detection thresholds. In contrast, at time T1 (after disease progression), sensitivity may worsen (e.g., greater skin indentation is needed for detection to occur). Still later, at time T2, sensitivity has decreased still further.

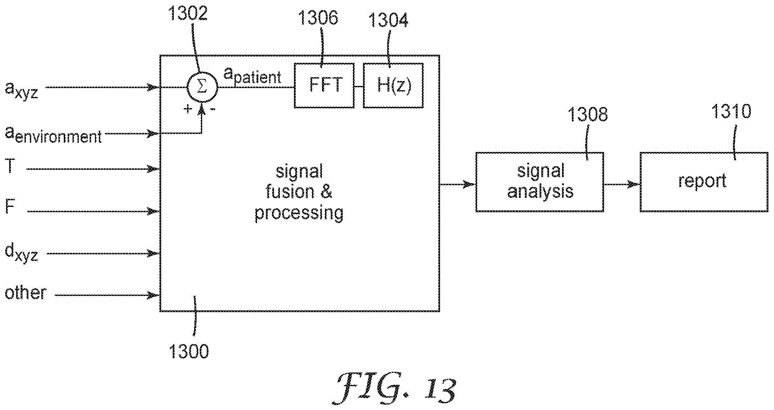

As mentioned earlier herein, especially during analysis and diagnosis of tremors, it can be important to account for environmental vibrations not related to tremors. Systems can be provided according to some embodiments to detect and account for such environmental vibrations. FIG. 13 illustrates signal flow from a vibration capture according to some embodiments. Inputs to signal processing 1300 can include the measured vibration or tremor $a_{xyz}$. In some implementations and test environments, environmental vibration can occur; for example, some vibration sources may be inherent in the human body (heart, lung, gut, etc.) and systems and methods according to embodiments can identify and account for these vibrations in subsequent analysis. Such environmental vibration $a_{environment}$ is therefore also provided to signal processing 1300. Other inputs include time of measured vibration T, frequency of measured vibration F, displacement $d_{xyz}$, and other signals.

A measure of vibration experienced by the patient ($a_{patient}$) can be calculated by removing the environmental vibration ($a_{environment}$) from that which is measured by an actuator assembly 106 or 206, noting as described above that $a_{patient}$ has an associated (x, y, z) reference coordinate system based on, for example, the direction of gravity, as well as an associated environmental vibration i.e., $a_{patient} = a_{xyz} - a_{environment}$. This is illustrated in FIG. 13 at summation 1302 (e.g., by subtracting the sampled time domain data). In alternative embodiments, subtraction can occur in the frequency domain using the fast Fourier transform (FFT) of the above-described vibration signals according to Equation (1):

$$F ( a_{patient} ) = F ( a_{xyz} ) - F ( a_{environment} ) \quad (1)$$

Signal processing and fusion may be used to signal process and enhance the various signal inputs for signal analysis. Examples of this processing and fusion may be signal averaging and application of frequency specific filters to remove, enhance the frequency content of the signal at block 1304. Signal analysis 1308 may include calculating the average frequency of tremor (found at block 1306). Variations in tremor frequency and intensity can also be determined. A report can be provided at 1310.

In some embodiments, processing circuitry 800 can calculate statics based on a tremor's data acquisition (e.g., based on $a_{xyz}$ vs. T data, as input to block 1300 above). Alternately, this data could be shown graphically as discrete data points (a sequence of numbers) occurring at discrete time samples. This sequence of numbers is divided into data frames. In each frame the processing circuitry 800 can calculate tremor frequency or power compare the result across a number of frames. Alternately, the processing circuitry 800 can generate a moving filter that moves continuously from t=0 (or n=0) to the end of the data and calculate a sliding average tremor frequency. Techniques to do the above include Fourier, wavelet or other transform methods. The frame data is compared in with data from the same session or from days, months or years earlier to compare tremor statistics.

Computing systems

FIG. 14 is a block diagram illustrating a machine in the example form of a computer system 1400, within which a set or sequence of instructions can be executed to cause the machine to perform any one of the methodologies discussed herein, according to an example embodiment. In some embodiments, different instantiations of the computer system 1400 can be used to implement all or parts of the functionality of the actuator assembly 106 (FIG. 1), clinician device 110, feedback device 114, or other element of system 100 (FIG. 1). In alternative embodiments, the machine operates as a standalone device or can be connected (e.g., networked) to other machines. In a networked deployment, the machine can operate in the capacity of either a server or a client machine in server-client network environments, or it can act as a peer machine in peer-to-peer (or distributed) network environments. The machine can be a personal computer (PC), a tablet PC, a hybrid tablet, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein.

Example computer system 1400 includes at least one processor 1402 (e.g., a central processing unit (CPU), a graphics processing unit (GPU) or both, processor cores, compute nodes, etc.), a main memory 1404 and a static memory 1406, which communicate with each other via a link 1408 (e.g., bus). The computer system 1400 can further include a video display unit 1410, an alphanumeric input device 1412 (e.g., a keyboard), and a user interface (UI) navigation device 1414 (e.g., a mouse). In one embodiment, the video display unit 1410, input device 1412 and UI navigation device 1414 are incorporated into a touch screen display. The computer system 1400 can additionally include a storage device 1416 (e.g., a drive unit), a signal generation device 1418 (e.g., a speaker), a network interface device 1420, and one or more sensors (not shown), such as a global positioning system (GPS) sensor, compass, accelerometer, or other sensor.

The storage device 1416 includes a non-transitory machine-readable medium 1422 on which is stored one or more sets of data structures and instructions 1424 (e.g., software) embodying or utilized by any one or more of the methodologies or functions described herein. The instructions 1424 can also reside, completely or at least partially, within the main memory 1404, static memory 1406, and/or within the processor 1402 during execution thereof by the computer system 1400, with the main memory 1404, static memory 1406, and the processor 1402 also constituting machine-readable media.

While the machine-readable medium 1422 is illustrated in an example embodiment to be a single medium, the term "machine-readable medium" can include a single medium or multiple media (e.g., a centralized or distributed database, and/or associated caches and servers) that store the one or more instructions 1424. The term "machine-readable medium" shall also be taken to include any tangible medium that is capable of storing, encoding or carrying instructions for execution by the machine and that cause the machine to perform any one or more of the methodologies of the present disclosure or that is capable of storing, encoding or carrying data structures utilized by or associated with such instructions. The term "machine-readable medium" shall accordingly be taken to include, but not be limited to, solid-state memories, and optical and magnetic media. Specific examples of machine-readable media include non-volatile memory, including, but not limited to, by way of example, semiconductor memory devices (e.g., electrically programmable read-only memory (EPROM), electrically erasable programmable read-only memory (EEPROM)) and flash memory devices; magnetic disks such as internal hard disks and removable disks; magneto-optical disks; and compact disc read-only memory (CD-ROM) and digital versatile disc-read-only memory (DVD-ROM) disks.

The instructions 1424 can further be transmitted or received over a communications network 1426 using a transmission medium via the network interface device 1420 utilizing any one of a number of well-known transfer protocols (e.g., hypertext transfer protocol (HTTP)). Examples of communication networks include a local area network (LAN), a wide area network (WAN), the Internet, mobile telephone networks, plain old telephone (POTS) networks, and wireless data networks (e.g., Wi-Fi, 3G, and 4G long term evolution (LTE)/LTE-Advanced (LTE-A) or WiMAX networks). The term "transmission medium" shall be taken to include any intangible medium that is capable of storing, encoding, or carrying instructions for execution by the machine, and includes digital or analog communications signals or other intangible medium to facilitate communication of such software.

Various modifications and alterations to this invention will become apparent to those skilled in the art without departing from the scope and spirit of this invention. It should be understood that this invention is not intended to be unduly limited by the illustrative embodiments and examples set forth herein and that such examples and embodiments are presented by way of example only with the scope of the invention intended to be limited only by the claims set forth herein as follows.

**Claims**

1. An apparatus for measuring peripheral nerve impairment, the apparatus comprising:

    a housing (300) that includes a mechanically compliant surface (500);
    an actuator (104) accessible from an outer surface of the housing (300) and in contact with the mechanically compliant surface of the housing (300), the actuator (104) being configured to transfer, via the mechanically compliant surface (500), a stimulation signal to a skin surface that is in contact with the housing (300); and
    processing circuitry (800) configured to:

        control the actuator (104) to generate the stimulation signal;
        receive, from a subject (102), a response to the stimulation signal; and
        based on the received response, form a diagnosis with respect to a neurological disability.

2. The apparatus of claim 1, further comprising an accelerometer (802), wherein to receive the response to the stimulation signal, the processing circuitry (800) is configured to receive a vibration measurement from the accelerometer (802).

3. The apparatus of claim 2, further comprising a memory, wherein the processing circuitry (800) is further configured to record, in the memory, the vibration measurement received from the accelerometer (802), and one or more of date information, time information, stimulation information, protocol information, or subject identification information, or subject response information associated with the received response.

4. The apparatus of any of claims 1-3, wherein the stimulation signal is a vibrotactile signal, and wherein the processing circuitry (800) is further configured to receive stimulation information that is based on at least one of an amplitude or a phase of the vibrotactile signal.

5. A system comprising:

a housing (300) that includes a mechanically compliant surface (500);
an actuator assembly (106) accessible from an outer surface of the housing (300) and in contact with the mechanically compliant surface (500) of the housing (300), the actuator assembly (106) including
an actuator (104) configured to transfer, via the mechanically compliant surface (500) of the housing (300), a stimulation signal to a skin surface that is in contact with the housing (300);
a clinician device (110);
processing circuitry (800) configured to control the actuator (104) to generate the stimulation signal;
a feedback device (114) configured to:

receive, from a subject (102), a response to the stimulation signal transferred to the skin surface; and
provide feedback to the clinician device (110) configured to:

provide test inputs to the processing circuitry (800) of the actuator assembly (106); and
receive, from the feedback device (114), respective responses to each respective test input, wherein the processing circuitry (800) is configured to receive, from the subject (102), a response to the stimulation signal.

6. The system of claim 5, wherein to transfer the stimulation signal, the actuator (104) is configured to transfer the stimulation signal primarily in an axis perpendicular to the mechanically compliant surface (500).

7. The system of claim 5, wherein the stimulation signal includes a vibration signal and wherein the processing circuitry (800) is further configured to record subject response information and stimulation information based on at least one of an amplitude or a phase of the vibration signal.

8. The system of any of claims 5-7, wherein the stimulation signal has a frequency, amplitude or phase as provided by a test protocol.

**Patentansprüche**

1. Eine Vorrichtung zum Messen einer peripheren Nervenbeeinträchtigung, die Vorrichtung aufweisend:

ein Gehäuse (300), das eine mechanisch nachgiebige Oberfläche (500) aufweist;
einen Aktuator (104), das von einer Außenoberfläche des Gehäuses (300) zugänglich ist und mit der mechanisch nachgiebigen Oberfläche des Gehäuses (300) in Kontakt steht, wobei der Aktuator (104) dazu eingerichtet ist, über die mechanisch nachgiebige Oberfläche (500) ein Stimulationssignal an eine Hautoberfläche zu übertragen, die mit dem Gehäuse (300) in Kontakt steht; und
Verarbeitungsschaltlogik (800), die dazu eingerichtet ist:

den Aktuator (104) zu steuern, um das Stimulationssignal zu erzeugen;
eine Antwort auf das Stimulationssignal von einem Subjekt (102) zu empfangen; und
basierend auf der empfangenen Antwort, eine Diagnose in Bezug auf eine neurologische Behinderung zu bilden.

2. Die Vorrichtung nach Anspruch 1, ferner aufweisend einen Beschleunigungsmesser (802), wobei, um die Antwort auf das Stimulationssignal zu empfangen, die Verarbeitungsschaltlogik (800) dazu eingerichtet ist, eine Vibrationsmessung von dem Beschleunigungsmesser (802) zu empfangen.

3. Die Vorrichtung nach Anspruch 2, ferner aufweisend einen Speicher, wobei die Verarbeitungsschaltlogik (800) ferner dazu eingerichtet ist, in dem Speicher die Vibrationsmessung, die von dem Beschleunigungsmesser (802) empfangen wird, und eine oder mehrere von Dateinformationen, Zeitinformationen, Stimulationsinformationen, Protokollinformationen oder Subjektidentifikationsinformationen oder Subjektantwortinformationen, die der empfangenen Antwort zugeordnet sind, aufzuzeichnen.

4. Die Vorrichtung nach einem der Ansprüche 1 bis 3, wobei das Stimulationssignal ein vibrotaktiles Signal ist und wobei die Verarbeitungsschaltlogik (800) ferner dazu eingerichtet ist, Stimulationsinformationen zu empfangen, die auf mindestens einem von einer Amplitude oder einer Phase des vibrotaktilen Signals basieren.

5. Ein System, aufweisend:

ein Gehäuse (300), das eine mechanisch nachgiebige Oberfläche (500) aufweist;
eine Aktuatoranordnung (106), die von einer Außenoberfläche des Gehäuses (300) zugänglich ist und mit der mechanisch nachgiebigen Oberfläche (500) des Gehäuses (300) in Kontakt steht, wobei die Aktuatoranordnung (106) aufweist
einen Aktuator (104), der dazu eingerichtet ist, über die mechanisch nachgiebige Oberfläche (500) des Gehäuses (300) ein Stimulationssignal an eine Hautoberfläche zu übertragen, die mit dem Gehäuse (300) in Kontakt steht;
eine Klinikarztvorrichtung (110);
Verarbeitungsschaltlogik (800), die dazu eingerichtet ist, den Aktuator (104) zu steuern, um das Stimulationssignal zu erzeugen;
eine Rückmeldungsvorrichtung (114), die dazu eingerichtet ist:

eine Antwort auf das Stimulationssignal, das auf die Hautoberfläche übertragen wird, von einem Subjekt (102) zu erhalten; und
eine Rückmeldung an die Klinikarztvorrichtung (110) bereitzustellen, die dazu eingerichtet ist:

Testeingaben an die Verarbeitungsschaltlogik (800) der Aktuatoranordnung (106) bereitzustellen; und
von der Rückmeldungsvorrichtung (114) jeweilige Antworten auf jede Testeingabe zu erhalten, wobei die Verarbeitungsschaltlogik (800) dazu eingerichtet ist, von dem Subjekt (102) eine Antwort auf das Stimulationssignal zu empfangen.

6. Das System nach Anspruch 5, wobei, um das Stimulationssignal zu übertragen, der Aktuator (104) dazu eingerichtet ist, das Stimulationssignal hauptsächlich in einer Achse senkrecht zu der mechanisch nachgiebigen Oberfläche (500) zu übertragen.

7. Das System nach Anspruch 5, wobei das Stimulationssignal ein Vibrationssignal aufweist und wobei die Verarbeitungsschaltlogik (800) ferner dazu eingerichtet ist, Subjektantwortinformationen und Stimulationsinformationen basierend auf einer Amplitude oder einer Phase des Vibrationssignals aufzuzeichnen.

8. Das System nach einem der Ansprüche 5 bis 7, wobei das Stimulationssignal eine Frequenz, Amplitude oder Phase umfasst, wie durch ein Testprotokoll bereitgestellt.

**Revendications**

1. Appareil permettant de mesurer une déficience des nerfs périphériques, l'appareil comprenant :

un boîtier (300) qui comporte une surface mécaniquement souple (500) ;
un actionneur (104) accessible à partir d'une surface externe du boîtier (300) et en contact avec la surface mécaniquement souple du boîtier (300), l'actionneur (104) étant conçu pour transférer, par l'intermédiaire de la surface mécaniquement souple (500), un signal de stimulation à une surface cutanée qui est en contact avec le boîtier (300) ; et
un circuit de traitement (800) conçu pour :

commander l'actionneur (104) pour générer le signal de stimulation ;
recevoir, à partir d'un sujet (102), une réponse au signal de stimulation ; et
en fonction de la réponse reçue, poser un diagnostic par rapport à un handicap neurologique.

**2.** Appareil selon la revendication 1, comprenant en outre un accéléromètre (802), dans lequel, pour recevoir la réponse au signal de stimulation, le circuit de traitement (800) est configuré pour recevoir une mesure de vibrations provenant de l'accéléromètre (802).

**3.** Appareil selon la revendication 2, comprenant en outre une mémoire, dans lequel le circuit de traitement (800) est en outre configuré pour enregistrer, dans la mémoire, la mesure de vibrations reçue de l'accéléromètre (802), et une ou plusieurs informations de date, informations d'heure, informations de stimulation, informations de protocole ou informations d'identification de sujet, ou informations de réponse de sujet associées à la réponse reçue.

**4.** Appareil selon l'une quelconque des revendications 1 à 3, dans lequel le signal de stimulation est un signal vibrotactile, et dans lequel le circuit de traitement (800) est en outre configuré pour recevoir des informations de stimulation qui sont basées sur au moins l'une parmi une amplitude ou une phase du signal vibrotactile.

**5.** Système comprenant :

un boîtier (300) qui comporte une surface mécaniquement souple (500) ;
un ensemble actionneur (106) accessible à partir d'une surface externe du boîtier (300) et en contact avec la surface mécaniquement souple (500) du boîtier (300), l'ensemble actionneur (106) comportant
un actionneur (104) conçu pour transférer, par l'intermédiaire de la surface mécaniquement souple (500) du boîtier (300), un signal de stimulation à une surface cutanée qui est en contact avec le boîtier (300) ;
un dispositif de clinicien (110) ;
un circuit de traitement (800) configuré pour amener l'actionneur (104) à générer le signal de stimulation ;
un dispositif de rétroaction (114) configuré pour :

recevoir, à partir d'un sujet (102), une réponse au signal de stimulation transféré à la surface cutanée ; et
fournir une rétroaction au dispositif de clinicien (110) configuré pour :

fournir des entrées de test au circuit de traitement (800) de l'ensemble actionneur (106) ; et
recevoir, à partir du dispositif de rétroaction (114), des réponses respectives à chaque entrée de test respective, dans lequel le circuit de traitement (800) est configuré pour recevoir, à partir du sujet (102), une réponse au signal de stimulation.

**6.** Système selon la revendication 5, dans lequel, pour transférer le signal de stimulation, l'actionneur (104) est configuré pour transférer le signal de stimulation principalement dans un axe perpendiculaire à la surface mécaniquement souple (500).

**7.** Système selon la revendication 5, dans lequel le signal de stimulation comporte un signal de vibration, et dans lequel le circuit de traitement (800) est en outre configuré pour enregistrer des informations de réponse de sujet et des informations de stimulation en fonction d'au moins l'une parmi une amplitude ou une phase du signal de vibration.

**8.** Système selon l'une quelconque des revendications 5 à 7, dans lequel le signal de stimulation a une fréquence, une amplitude ou une phase telle que fournie par un protocole de test.

*FIG. 1*

EP 3 975 826 B1

*FIG. 2*

FIG. 3

FIG. 4

*FIG. 5*

Mechanically compliant window

Vibrotactile Actuator

*FIG. 6*

Infection barrier

Removal tab

Mechanically compliant window

*FIG. 7*

*FIG. 8*

EP 3 975 826 B1

900

```
┌─────────────────────────────────┐
│  Provide vibrotactile  stimulation  │── 902
└─────────────────────────────────┘
                 │
                 ▼
┌─────────────────────────────────┐
│  Receive an indication  as to whether │── 904
│  the stimulation  was sensed        │
└─────────────────────────────────┘
                 │
                 ▼
┌─────────────────────────────────┐
│  Analyze the indication  to diagnose a │── 906
│  neurological  disability            │
└─────────────────────────────────┘
```

*FIG. 9*

1000

```
┌─────────────────────────────────┐
│  Provide an accelerometer  in physical │── 1002
│  contact with  a limb or digit  of a   │
│  subject                            │
└─────────────────────────────────┘
                 │
                 ▼
┌─────────────────────────────────┐
│  Receive a movement  indication  at  │── 1004
│  the accelerometer                  │
└─────────────────────────────────┘
                 │
                 ▼
┌─────────────────────────────────┐
│  Analyze the indication  to diagnose a │── 1006
│  neurological  disability            │
└─────────────────────────────────┘
```

*FIG. 10*

*FIG. 11*

*FIG. 12*

a$_{xyz}$

a$_{environment}$

T

F

d$_{xyz}$

other

1302

Σ

+ −

a$_{patient}$

1306 FFT

1304 H(z)

1300 signal fusion & processing

1308 signal analysis

1310 report

*FIG. 13*

1400

1402 — PROCESSOR
1424 — INSTRUCTIONS

1410 — VIDEO DISPLAY

1404 — MAIN MEMORY
1424 — INSTRUCTIONS

1412 — ALPHA-NUMERIC INPUT DEVICE

1408

1406 — STATIC MEMORY

1414 — UI NAVIGATION DEVICE

BUS

1416
DRIVE UNIT
1422 — MACHINE-READABLE MEDIUM
1424 — INSTRUCTIONS

1420 — NETWORK INTERFACE DEVICE

1426 — NETWORK

1418 — SIGNAL GENERATION DEVICE

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012220892 A1

- US 5002065 A

**Non-patent literature cited in the description**

- Standardised method of determining vibratory perception thresholds for diagnosis and screening in neurological investigation. **GOLDBERG J M et al.** JOURNAL OF NEUROLOGY NEUROSURGERY & PSYCHIATRY. BMJ PUBLISHING GROUP, 01 September 1979, vol. 42